# EUROPEAN PATENT APPLICATION

(11) **EP 2 206 490 A2**
(43) Date of publication of application: **14.07.2010**
(21) Application number: 10161302.4
(22) Date of filing: 28.07.2006
(51) Int. Cl.: A61K 8/02, A61K 9/00, A61K 9/70, A61Q 11/00

(54) **Adherent and erodible film to treat a moist surface of a body tissue**

(30) Priority: 29.07.2005 US 192524
(62) Divisional of application: 06788856.0
(71) Applicant: Uluru Inc., Addison, TX 75001 (US)
(72) Inventor: Moro, Daniel G., Carrollton, TX 75007 (US); Nowotnik, David P., 76034, TX Colleyville (US); Callahan, Ernest H. Jr., North Richland Hills, TX 76180 (US)
(74) Representative: Viering, Jentschura & Partner

(57) **Abstract**

A thin, flexible, bilayer or multi-layer film which when applied to a moist surface of a body tissue adheres and delivers an active agent, pharmaceutical compound, nutraceutical, flavor or other substance to the underlying surface and/or body cavity and erodes at a predetermined rate. In one application, the amount of time that the active agent remains in contact with the teeth surfaces is controlled by the composition and thickness of the backing layer of the composite film. This erosion or residence time can be regulated from one half hour to several hours, depending upon the desired therapeutic or cosmetic application.

## Description

### FIELD OF THE INVENTION

This invention relates to the cosmetic or therapeutic treatment of teeth, gums or mucosal surfaces and more specifically to an adherent, erodible film that provides one or more active agents, pharmaceutical compounds, neutraceutical compounds, cosmeceutical compounds, vitamins, minerals, botanical extracts, flavors, fragrances or other substances to teeth, oral cavity and other mucosal surfaces for a prolonged and controlled period of time.

### BACKGROUND OF THE INVENTION

A tooth is composed of a protective, hard enamel outer layer and an inner dentin layer. The enamel layer is typically opaque white or slightly off-white in color.

This layer is composed of hydroxyapatite mineral crystals and is somewhat porous, allowing staining agents and discoloring substances to permeate the enamel and discolor teeth. In particular, certain foods, tobacco products and liquids such as tea and coffee tend to stain teeth. These substances accumulate on the surface and form a film on the teeth, and will then permeate into the enamel layer. This problem occurs over many years, imparting a noticeable discoloration of the enamel layer.

There have been numerous methods in the prior art relating to teeth whitening, including brushing the teeth using dentifrices containing an effective oxidizing agent such as peroxide. These types of compositions are disclosed in US Pat. No. 5,256,402. More recently, several over-the-counter teeth whitening systems have become available and have gained in popularity as an alternative cosmetic treatment to teeth whitening procedures conducted by a professional. One such product is comprised of a thin strip of plastic film that has a tooth whitening composition applied to the surface as described in US Pat. Nos.5,894,017, 5,891,453 and 6,045,811. In addition, US Pat No. 6,419,906 B1 describes a flexible film which when applied to stained teeth is hydrated by saliva and is effective in such form to whiten teeth. The film comprises an anhydrous water hydratable ethylene oxide polymer matrix containing a solid peroxide whitening agent whereby upon placing and positioning on stained teeth, the peroxide is solubilized and activated by the saliva present in the oral cavity. For example, US Patent Appl. Publ. No. 2003/0133884A1 discloses a dry tooth-whitening patch in which a tooth-whitening agent is dispersed throughout a matrix type adhesive layer. The patch also contains an impermeable backing layer on the outside surface. This system provides a superior whitening affect, due to the improved adhesion to a wet tooth surface.

All of these aforementioned systems produce a whitening effect when applied to stained teeth; however, the strips must be removed after a specified period of time. The invention disclosed herewith overcome this limitation and provide other advantages as well.

### SUMMARY OF THE INVENTION

This invention provides an erodible, multi-layered device that adheres to a moist surface of a body tissue, such as the oral mucosa, gum, tongue, tooth enamel, and vaginal mucosa. In one embodiment, the device provides the controlled delivery of an active agent to a moist surface of a body tissue. The device contains a backing layer for controlling the residence time of a water-soluble thin-film device that is adhered to a moist surface of a body tissue. In one aspect, the backing layer is an erodible film composed of both hydrophobic and hydrophilic polymers. In a separate aspect, the backing layer is erodible comprising at least one hydrophobic polymer and at least one water-soluble polymer wherein the ratio of the hydrophobic polymer(s) to the water soluble polymer(s) is from 1:1 to 9:1 by weight. In another aspect, the water-erodible backing layer further comprises a plasticizer or other agent that affects the erosion rate. In yet a further aspect, the water-erodible backing layer comprises a flavor or masking agent to mitigate the taste of the active compound.

One object of the present invention is to provide a novel, cost-effective, erodible, layered device that adheres to the moist surfaces of teeth and delivers an active agent for a controlled period of time. Another object of the present invention is to provide a convenient, user-friendly, erodible, layered device that adheres to moist teeth surfaces and delivers a tooth-whitening agent to the underlying stained surfaces. A further object of this invention is to provide a tooth-whitening device that is easily applied without breaking or leaving any unwanted residue on the hands. Another object of the present invention is to provide a flexible, layered device that conforms and adheres intimately and securely to the entire tooth surface, minimizing the exposure of any excess whitening agent to the surrounding gums as found with other competitive products.

In one aspect, the composition of the bi-layered and multi-layered devices consists of an enamel adherent, water soluble, polymeric layer containing a tooth whitening agent or other active compound and a coated, erodible backing layer that controls the desired residence time. Since the devices of the current invention can provide a more effective contact time with a stained tooth surface before eroding, it is expected that lower and safer amounts of whitening agents can be used to accomplish similar or superior results than attained by other commercial products.

Another aspect of the present invention is an erodible multilayered strip comprising at least two layers, a first layer comprises a water soluble polymer or combination of polymers that adheres to moist enamel surfaces. A second layer is water erodible and controls the residence time that the strip remains adhered to the enamel surface. This erodible strip is preferably shaped to conform to an individual tooth or a row of teeth.

One embodiment of the present invention is an adherent, erodible multi-layered device comprising a first, water-soluble adhesive layer to be placed in contact with a moist surface of a body tissue, and a second, water-erodible non-adhesive backing layer that controls residence time of the device. The first layer comprises a tooth-whitening agent or other active, at least one water-soluble film-forming polymer, in combination with at least one mucoadhesive polymer if the application is directed to mucosal applications; and said second, water-erodible non-adhesive backing layer comprises a precast film containing at least one of hydroxypropyl methylcellulose, hydroxyethyl cellulose, hydroxypropyl cellulose, polyvinyl alcohol, polyethylene glycol, polyethylene oxide, and ethylene oxide-propylene oxide co-polymer, said backing layer being coated with at least one hydrophobic polymer, alone or in combination with at least one hydrophilic polymer, such that the backing layer is bioerodible.

Another aspect of the present invention is an adherent, erodible multi-layered device comprising a first, water-soluble adhesive layer to be placed in contact with a moist surface of a body tissue, and a second, water-erodible non-adhesive backing layer that controls residence time of the device. In one aspect, the first layer comprises a tooth-whitening agent or other active, at least one water-soluble film-forming polymer, in combination with at least one mucoadhesive polymer if the application is directed to mucosal applications; and said second, water-erodible non-adhesive backing layer comprises a coating of at least one hydrophobic polymer, alone or in combination with at least one hydrophilic polymer, such that the backing layer is bioerodible.

A yet further aspect of the present invention is an adherent, erodible multi-layered device comprising a first, water-soluble adherent layer to be placed in contact with a moist surface of a body tissue, a second, water-soluble layer and a third, water-erodible non-adhesive layer that controls the residence time of the device. The first layer comprises at least one water-soluble film-forming polymer, in combination with at least one mucoadhesive polymer if the application is directed to mucosal applications; and said second, water-soluble layer preferably comprises a tooth-whitening agent or other active in combination with at least on water-soluble film-forming polymer; and said third, water-erodible non-adhesive backing layer comprises a precast film containing at least one of hydroxypropyl methylcellulose, hydroxyethyl cellulose, hydroxypropyl cellulose, polyvinyl alcohol, polyethylene glycol, polyethylene oxide, and ethylene oxide-propylene oxide co-polymer, said backing layer being coated with at least one hydrophobic polymer, alone or in combination with at least one hydrophilic polymer, such that the backing layer is bioerodible. This combination of layers is utilized for those applications in which the presence of an active or other non-pharmaceutical agent impairs the adhesive characteristics of the adherent layer and thus a first adhesive layer is required to improve the bonding efficiency between the multilayered film and a moist surface of a body tissue.

The present invention also provides an adherent, erodible multi-layered device comprising a first, water-soluble adhesive layer to be placed in contact with a moist surface of a body tissue, a second, water-soluble layer and a third, water-erodible non-adhesive layer that controls the residence time of the device. The first layer comprises at least on water-soluble film-forming polymer, in combination with at least one mucoadhesive polymer if the application is directed to mucosal applications; and said second, water-soluble layer preferably comprises a tooth-whitening agent or other active in combination with at least one water-soluble film-forming polymer; and said third, water-erodible non-adhesive backing layer comprises a coating of at least one hydrophobic polymer, alone or in combination with at least one hydrophilic polymer, such that the backing layer is bioerodible.

The present invention provides an adherent, erodible multi-layered device comprising a first, water-soluble adhesive layer to be placed in contact with a moist surface of a body tissue, and a second, water-erodible non-adhesive backing layer that controls residence time of the device. The first layer comprises a tooth-whitening agent, preferably urea peroxide, hydrogen peroxide, polyvinyl pyrrolidone/hydrogen peroxide complex or sodium percarbonate, and most preferably sodium percarbonate, or other active, and at least one water-soluble film-forming polymer in combination. Said second, water-erodible non-adhesive backing layer comprises a precast film containing at least one of hydroxypropyl methylcellulose, hydroxyethyl cellulose, hydroxypropyl cellulose, polyvinyl alcohol, polyethylene glycol, polyethylene oxide, and ethylene oxide-propylene oxide co-polymer. This backing layer is coated with at least one hydrophobic polymer, alone or in combination with at least one hydrophilic polymer, such that the backing layer is bioerodible. The second water-erodible non-adhesive backing layer can act as a casting and support surface if desired on which the adhesive layer is prepared. In one aspect, the device comprises a premade film of hydroxypropyl methyl cellulose in combination with a coating consisting of at least one hydrophobic polymer selected from the family of quaternary ammonium acrylate/methacrylate co-polymers, (Eudragit RS) ethyl cellulose and methyl cellulose, alone or in combination with at least one hydrophilic polymer, selected from the group consisting of polyvinyl pyrrolidone, hydroxypropyl methylcellulose, hydroxyethyl cellulose, hydroxypropyl cellulose, and polyvinyl alcohol. Alternatively, the adherent layer can be produced directly on a release substrate known in the art, such as coated paper, polyethylene, polypropylene, mylar and the like, and the second water-erodible non-adhesive backing layer comprising a precast film containing at least one of hydroxypropyl methylcellulose, hydroxyethyl cellulose, hydroxypropyl cellulose, polyvinyl alcohol, polyethylene glycol, polyethylene oxide, and ethylene oxide-propylene oxide co-polymer is laminated to the surface of the first layer using a polymeric binding solution. This backing layer can be coated with at least one hydrophobic polymer in combination with at least one hydrophilic polymer, such that the backing layer is bioerodible. It is understood that, depending upon the application, multiple layers with or without active or therapeutic agents or combinations thereof, or other compounds previously mentioned can be laminated together forming the multilayered adherent erodible delivery devices described in this disclosure, and the resulting erosion rate is primarily controlled by the backing coating layer composition. In addition, varying the thickness of the backing coating layer will also affect the erosion rate. The thickness of the backing coating layer ranges from about 0.05 microns to about 100 microns, or alternatively from about 1.0 microns to about 50 microns, or alternatively less than 100 microns, or alternatively, less than 50 microns, or alternatively, from about 2 to about 50 microns or alternatively, from about 5 to about 20 microns.

### DETAILED DESCRIPTION OF THE EMBODIMENTS

### Definitions

As used in the specification and claims, the singular form "a", "an" and "the" include plural references unless the context clearly dictates otherwise. For example, the term "a layer" includes more than one layer.

As used herein, the term "comprising" is intended to mean that the compositions and methods include the recited elements, but not excluding others. "Consisting essentially of" when used to define compositions and methods, shall mean excluding other elements of any essential significance to the combination. "Consisting of" shall mean excluding more than trace elements of other ingredients and substantial method steps for using the devices of this invention. Embodiments defined by each of these transition terms are within the scope of this invention.

All numerical designations, e.g., pH, temperature, time, concentration, and molecular weight, including ranges, are approximations which are varied ( +) or (-) by increments of 0.1. It is to be understood, although not always explicitly stated that all numerical designations are preceded by the term "about". It also is to be understood, although not always explicitly stated, that the reagents described herein are merely exemplary and that equivalents of such are known in the art.

As used herein, a "surfactant" has the meaning generally understood by those skilled in the chemical art. That is, a surfactant is a soluble compound, which may be anionic, cationic, zwitterionic, amphoteric or neutral in charge, and which reduces the surface tension of the liquid in which it is dissolved or that reduces interfacial tension between two liquids or a liquid and a solid.

As used herein, the term "monomer" has the meaning understood by those skilled in the chemical art. That is, a monomer is a small chemical compound that is capable of forming a macromolecule of repeating units of itself, i.e., a polymer. Two or more different monomers may react to form a polymer in which each of the monomers is repeated numerous times, the polymer being referred to as a copolymer to reflect the fact that it is made up of more than one monomer.

As used herein, an "active agent" refers to any substance that is other than a carrier or excipient. Examples of active agents include, for example, biomedical agents; biologically active substances such as pharmaceutical agents, genes, proteins, growth factors, monoclonal antibodies, fragmented antibodies, antigens, polypeptides, DNA, RNA and ribozymes; agrichemical agents (herbicides, fungicides, insecticides, plant growth hormones, etc.); radiopaque substances; radioactive substances, pigments; dyes; metals; semiconductors; dopants; chemical intermediates; acids; and, bases. An additional example is a "pharmaceutical agent" which refers to both small molecule and to macromolecular compounds used as drugs. Among the former are, without limitation, antibiotics, chemotherapeutics (in particular platinum compounds and taxol and its derivatives), analgesics, antidepressants, anti-allergenics, anti-arryhthics, anti-inflammatory compounds, CNS stimulants, sedatives, anti-cholinergics, anti-arteriosclerotics, and the like. Macromolecular compounds include, without limitation, monoclonal antibodies (mAbs), monoclonal antibody fragments (Fabs), proteins, peptides, cells, antigens, nucleic acids, enzymes, growth factors and the like. A pharmaceutical agent may be intended for topical or systemic use.

As used herein, a "cross-linking agent" refers to a di-, tri-, or tetrafunctional chemical entity that is capable of forming covalent bonds with functional groups on polymeric strands resulting in a three-dimensional structure.

As used herein, the term *"ex vivo*" refers to any process or procedure being performed outside of a living organism, for instance, without limitation, in a Petri dish, in soil, in surface water, in a liquid organic medium and the like.

As used herein, the term "*in vivo*" refers to any process or procedure performed within a living organism, which may be a plant or an animal, in particular, in a human being.

As used herein, the term "polymer" shall mean a high-molecular weight substance made up by the repetition of some simpler unit, for example, the monomer.

The term "hydrophobic polymer" shall mean any polymer that has little or no affinity for water, does not imbibe water and is not soluble or dispersible in water without the aid of a solubilizing agent.

The term 'water-soluble polymer" shall mean any polymer that has affinity for water, is soluble or uniformly dispersible in water without the aid of a solubilization agent.

As used herein, the term "water-erodible coating layer" shall mean a distinct layer composed of hydrophobic and water-soluble polymers in a specific ratio such that upon exposure to physiological body fluid, e.g., saliva, the water- soluble polymer dissolves from the layer leaving behind a weakened layer that eventually erodes away due to mechanical action.

"Residence Time" is the time the multi-layered device remains on a moist surface of a body tissue until complete erosion.

A "plasticizer" is additive that softens and imparts flexibility to a rigid material, typically a polymer, by swelling the amorphous region and lowering the cohesion between the polymer chains.

A "humectant" is a substance that promotes the retention of water.

The term "a coloring agent" shall mean a substance that imparts color or changes the tint or shade of an existing color of a material.

An "opacifying agent" is a substance that reduces the clarity of a material.

A "taste-masking agent" is a substance that is added to a formulation to mask or reduce the unpleasant taste of an active ingredient by promoting a powerful palatable oral sensory-directed reaction.

"Mechanically bonded" as used herein shall mean a physical bond accomplished by such means of promoting interlocking macromolecular filaments that are not chemically (ionically or covalently) bonded, through hydrophobic and/or hydrophilic interactions at the surface.

A "nutraceutical" is a substance that improves a product's heath attributes for the expressed purpose of treatment or prevention of disease.

A "cosmeceutical" is a cosmetic that contains biologically active ingredients that claims to have medical benefits.

As used herein, the term "average molecular weight" refers to the weight of individual polymer strands or cross-linked polymer strands of this invention. For the purpose of this invention, average molecular weight is determined by gel permeation chromatography with laser light scattering detection.

As used herein, the symbol "µ" is the abbreviation for micron or micrometer, or µm.

### Detailed Description

The present invention provides a unique, erodible, layered device that adheres to a moist surface of a body tissue. In one aspect, the invention provides a water-erodible device that adheres to gums and tooth enamel surfaces. In another aspect, the invention provides a water-erodible, multilayered device that adheres to mucosal surfaces and erodes at a controlled rate, delivering an active or non-active pharmaceutical compound to the underlying surface and/or the oral cavity.

The device is applicable for the cosmetic treatment of stained teeth, by delivering a whitening agent or combination of agents thereof for a controlled period of time. The device can also be used to deliver fluoride ions and phosphates for the preventative treatment of caries and tartar accumulation, respectively or other actives known to treat other diseases in the oral cavity. In addition, the device can be designed to contain specific, reactive compounds in different layers comprising the film, such that upon application to a tooth, gum or mucosal surface, the compounds combine forming a new chemical entity to treat a specific disorder or cosmetic application. One example of this type of device would be for the mineralization of tooth surface, by incorporating calcium ions in one layer and phosphate ions in another layer, such that they will combine upon application of the composite film to a moist tooth surface and exposure to saliva to form amorphous calcium phosphate and eventually apatite, which is very similar to tooth mineral.

The versatility of the device allows it to be used therapeutically on mucosal surfaces by simple incorporation of a mucoadhesive polymer in the adherent layer and a pharmaceutical in one or more layers of the multi-layered device. The erosion time for all of these devices is controlled by the backing layer, its composition and thickness, and is also affected by the overall thickness of the device. The erosion rate can be varied from about fifteen minutes to approximately three hours depending upon the specific therapeutic or cosmetic application.

The device initially adheres to a moist tooth surface due to the hydration and partial solubilization of the water-soluble polymer layer. The whitening agent that is dispersed throughout this polymeric layer is then activated as it comes in contact with saliva and is released to the underlying surface. The erosion rate of the device is controlled by the coated backing layer, which affects the amount of time the whitening agent remains in contact with the enamel surface. The backing layer slows down the dissolution of the underlying water-soluble polymeric layer containing a whitening agent, and therefore maximizing the direct contact time and unidirectional delivery to the tooth surface. The composition of the backing layer is easily adjusted to provide variable erosion rates from fifteen minutes to several hours by altering the ratio of hydrophic: water-soluble polymer content and amount comprising the backing layer. The higher the ratio, the longer the erosion rate of the layered device, keeping the backing layer coating thickness and thickness of the adhesive layer of the device constant. The layered device is essentially totally erodible, and therefore does not require removal after the appropriate treatment time. Alternatively, the device can contain an adherent layer without any active agent, to improve the adhesion to the tooth surface. The second layer can contain the tooth-whitening agent dispersed throughout the water-soluble adhesive layer, and the third layer, the coated backing layer, as previously stated, controls the erosion rate of the laminated, multilayered device. In this case, the adherent layer immediately hydrates, allowing the diffusion of water into the second layer and activates the tooth-whitening agent. The liberated active agent then passes through the adherent layer to the tooth surface and whitens the tooth surface. As the second layer hydrates, it also becomes adherent and remains in place on the tooth surface while the treatment continues.

The Residence Time as previously defined is difficult to quantitatively ascertain. One visual method is to apply the layered device to the teeth surface and periodically observe the covered surface using a mirror and assess approximately how much residue remains on the surface. Typically, as the backing layer erodes away at a predetermined rate, the adhesive swells and starts to dissolve and fall off the teeth surface. The actual residence time on each surface is controlled primarily by the flow of saliva to the surface and any friction created by interaction with the internal surface of the lips.

The polymeric coating layer that adheres to the tooth enamel is composed of one or more adhesive polymers, an appropriate whitening agent and a plasticizer. This coating may also contain an antioxidant, a preservative, a flavor and a taste-masking compound.

The adhesive polymers can be any water-soluble, FDA approved polymer for oral applications that sticks to an enamel surface when in contact with a moist tooth surface. Examples of adhesive polymers include, but are not limited to hydroxyethyl cellulose, hydroxypropyl cellulose, hydroxypropylmethyl cellulose, hydroxyethylmethyl cellulose, sodium carboxymethyl cellulose, polyvinyl pyrrolidone, polyvinyl alcohol, polyethylene glycol, polyacrylic acid, polyethylene oxide, alone or in combination thereof. In one aspect, the polymers are hydroxyethyl cellulose and polyvinyl pyrrolidone since they exhibit rapid and effective adhesion to enamel when in contact with a moist tooth surface.

The molecular weight of the adhesive polymer is also important, since it must be large enough so that an integral film can form, but not so large that immediate interfacial solubilization and adhesion to the enamel surface is impaired. Typical average molecular weights range between about 50,000 and about 1,500,000 Daltons, or alternatively between about 50,000 and 1,000,000 Daltons, or alternatively between about 50,000 and 500,000 Daltons or alternatively between about 100,000 and 500,000 Daltons or alternatively between about 150,000 and 400,000 Daltons.

The weights of the embodiments detailed in this application depend on the specific end use. The weight of a laminated film suitable for a tooth whitening application that would cover at least six to eight teeth ranges from about 0.01 gram to about 10 gram, or alternatively, from about 0.10 gram to about 1.0 gram or alternatively, from about 0.2 gram to about 0.50 gram. The weights of a multilayered film for an oral mucosal, gum application or extended breath freshener product range from about 0.01 gram to about 5 grams, or alternatively less than about 5 grams, or alternatively, less than about 4 grams, or alternatively, from about 0.02 grams to about 1 gram and alternatively from about 0.025 gram to about 0.30 gram.

Whitening agents suitable for the practice of the present invention include but are not limited to peroxides, metal chlorites, perborates, percarbonates, peroxyacids, persulfates, pyrophosphates alone or in combination thereof. Suitable peroxide compounds include hydrogen peroxide, carbamide peroxide, calcium peroxide, and mixtures thereof. The peroxide can be carbamide peroxide. Suitable metal chlorites include but are not limited to calcium chlorite, barium chlorite, magnesium chlorite, lithium chlorite, sodium chlorite, and potassium chlorite. The chlorite can be sodium chlorite. A percarbonate can be sodium percarbonate and the persulfates are oxones. The pyrophosphates are sodium acid pyrophosphate and potassium pyrophosphate.

The rate at which the whitening agent is solubilized and subsequently released to a tooth surface is controlled by varying the film thickness, polymer properties such as hydrophilicity, structure and molecular weight, type and properties of whitening agent and the concentration of the whitening agent. The concentration of the whitening agent typically varies from about 0.1 % to about 30% by weight of the total layered device, or alternatively from about 0.5% to about 20% by weight.

A plasticizer useful for purposes of the present invention is a glycol selected from the group consisting of propylene glycol, polyethylene glycol, polyhydric alcohols such as glycerin and sorbitol and glycerol esters such as glycerol triacetate. The plasticizer comprises about 0.2% to about 30% by weight of the film of the present invention and alternatively about 0.5% to about 10% by weight. In another aspect, the plasticizer comprises from about 0.5% to about 9% by weight, or alternatively, from about 1.0% to about 8% by weight, or alternatively, at least 0.2%, or alternatively at least 0.5%, or alternatively 0.7%, or alternatively, at least 1.0%, each by weight.

Glycerin and propylene glycol are plasticizers for use in the present invention as well as polyethylene glycol. The molecular weights for polyethylene glycol are in the range of 200-600 Daltons.

A colorant or opacifier can be incorporated in the adhesive layer or any of the layers of this device for use as an appearance enhancer. The colorant or opacifier comprises from about 0.01 % to about 10% by weight of the film of the present invention or alternatively from about 0.03% to about 1% by weight. Colors and opacifiers may also be used to help distinguish the non-adhesive backing layer from the enamel adhering layer. Suitable opacifiers for use in this inventin include, but are not limited to titanium dioxide, zinc oxide, and zirconium silicate.

In addition to the incorporation of whitening agents, a plasticizer, and colorants, there may also be included in the adhesive film matrix a minor amount, e.g., from about 0.01 to about 2% by weight, of ingredients such as preservatives, antioxidants, and flavors.

The backing layer solution is composed of a mixture of a hydrophobic polymer, such as ethyl cellulose, methyl cellulose, propyl cellulose or other related polymers and copolymers, anionic, cationic and neutral polymers and copolymers of methyl methacrylate under the trade name EUDRAGIT®, and a water soluble polymer such as polyvinyl pyrrolidone, hydroxypropylmethyl cellulose, hydroxyethyl cellulose, hydroxypropyl cellulose, polyvinyl alcohol or any other water soluble polymer that can be completely commixed with the hydrophobic polymer(s), dissolved in ethanol or other suitable organic solvent. The ratio of the hydrophobic to the hydrophilic polymer is adjusted to increase or decrease the desired residence time that the device remains on the teeth before complete erosion. The ratio of hydrophobic to hydrophilic polymer ranges from about 1.0:1.0 to about 9:1 by weight or alternatively from about 1.0:1.0 to about 3:1 by weight. In further embodiments, the ratios can range from about 2.0:1.0, or alternatively, 3.0:1:0, or alternatively 4.0:1.0, or alternatively about 5.0:1.0 or alternatively about 6.0:1.0, or alternatively about 7.0:1.0, or alternatively about 8.0:1.0. It is understood that many other hydrophobic polymers or compounds known in the art, such as modified silicones, can be used in combination with a hydrophilic polymer to provide the required residence time. The only requirements are that these materials are FDA approved, can be easily commixed with the hydrophilic polymer and are dispersible in the appropriate solvent that provides a homogeneous backing layer solution or suspension prior to producing a film. If necessary, a suitable surfactant can be utilized to insure that a uniform coating suspension exists.

The backing layer solution may also contain a plasticizing agent, such as propylene glycol, polyethylene glycol, or glycerin, in a small amount, from less than about 2%, or alternatively from about 0.1 to about 2.0% by weight, in order to improve the flexibility and conformability of the resultant layered film and to adjust the erosion rate of the device. Colors and opacifiers may also be added to help distinguish the non-adhesive backing layer from the enamel adhering layer.

The backing layer solution may also contain a taste-masking agent or flavor, in a small amount, 0.1 to 5.0% by weight, in order to mitigate the taste of the active agent. The backing layer solution may contain a larger amount of flavor, up to 25 per cent by weight, if the application is directed to a long-acting breath freshener. A flavor would also be present in various amounts throughout the multi-layered film, up to 25 per cent in one or each layer comprising the device, and the coated backing layer would provide sustained release of the flavor over an extended period of time by regulating the erosion rate of the composite film.

Once dissolved or dispersed, these solutions or suspensions (adhesive and backing) are cast and processed into a thin film by techniques known in the art, such as by film dipping, film coating, film casting, spin coating, or spray drying. These films are produced on an appropriate support at the desired thickness and dried using an oven. The primary support can be a polymer-coated paper, Mylar or any other appropriate non-deformable and impervious surface. The preferable primary support is coated paper. The primary support's casting surface must hold the film dimensionally stable during the coating and drying processes, but allow the resulting composite film to release when desired. The actual casting surface for these solutions may be the primary support or another layer of the device being produced, such as the precast film layer or a freshly cast adhesive or backing layer. The amount of coating solutions applied, using a suitable doctor blade or lab coater apparatus, is less than about 1.5 mm *or* alternatively ranges between about 0.01 to about 1.5 mm, and alternatively less than about 0.4 mm or alternatively between about 0.05 and about 0.4 mm for the backing layer and less than about 1.3 mm or alternatively between about 0.8 and about 1.3 mm for the adhesive layer. The amount of solids present in the coating solutions, the resulting solution viscosity and coating thickness applied determine the amount of coating film to be deposited on the casting surface.

The final layered device will consist of an adhesive layer and a hydrophobic layer with or without a precast hydroxypropylmethyl cellulose (HPMC) film in-between. A precast water-soluble film between the adhesive and backing layers will typically provide a longer residence time on the teeth. Several methods of forming this device are now provided herein.

In one embodiment, the backing layer is formed on an appropriate primary support. A precast film of HPMC is then laminated to the backing layer using binding solution consisting of polyvinylpyrrolidone dissolved in a water/ethanol mixture. Then a distinct binding layer, typically composed of polyvinylpyrrolidone is formed on top of the HPMC precast film. Finally, the adhesive layer is formed on top of the binding layer. This resulting multi-layer film is then further processed as outlined above.

In another embodiment, the adhesive layer is formed on an appropriate primary support by the casting methods previously described. The backing layer is then formed directly on top of the adhesive layer. This two-layer film is then further processed as outlined above.

In another embodiment, the backing layer is formed on an appropriate primary support by the casting methods previously described. The adhesive layer is then formed directly on top of the backing layer and the resulting bilayered product is further processed as outlined above.

In one embodiment, the adhesive layer is formed on an appropriate primary support. In a separate operation, the backing layer is formed on a water soluble, polymeric precast film of HPMC. The two films are then laminated, with the hydrophobic layer either on the outside or in the middle of the final composite film, using an appropriate binding solution such as polyvinylpyrrolidone dissolved in a water/ethanol mixture. This multi-layered film can be either peeled away from the primary support and cut into the desired shape or cut with the primary support still attached.

The thicknesses of each layer will affect the residence time of the device. The hydrophobic layer composition and thickness are the most important parameters in controlling the residence time. However, inclusion of the water soluble, polymeric precast film as one of the layers of the device will also increase the residence time, since the overall thickness of the device is increased. The versatility of this invention allows one to easily adjust the residence time of the device. Increasing the ratio of hydrophobic:water-soluble polymer in the backing layer coating and also increasing the solids level present in this layer will provide a longer residence time. Conversely, reducing this ratio and amount of solids will reduce the residence time. Increasing the coating thickness of the backing coating layer will also extend the residence time in addition to producing a multilayered product with increased thickness. However, the most dominating variable in affecting the residence time is the composition and amount of hydrophobic and water-soluble polymers present in the backing coating layer.

The total thickness of the device is also an important consideration in regards to user acceptance. A very thick device becomes more noticeable with respect to "mouth feel", and may cause the user to discontinue its use prematurely, thus compromising efficacy.

The total thickness of the device will also affect its ability to conform and adhere to teeth in an efficient manner. Typically, thin films are preferred since they can be easily applied and bent around the teeth, minimizing the amount of unattached area in the form of edges and corners that could cause the film to be accidentally pulled off the teeth. However, if the film is too thin, it will begin to lose tensile strength and rip during its application.

With respect to the individual layers of the composite film for most applications, the thickness of the adhesive layer is between 50µ and 300µ, and alternatively between 60µ and 140µ. The thickness of the backing layer is between 50µ and 300µ, and alternatively between 60µ and 100µ. The thickness of the precast layer if used to produce a multilayered device is between 25µ and 200µ, and alternatively between 50µ and 100µ. The overall thickness of the device is between 75µ and 500µ, and alternatively between 125µ and 300µ. However, it is understood and within the scope of this invention, that devices requiring a longer residence time, such as an extended breath freshener, can be thicker than the aforementioned ranges.

The following examples are intended to illustrate, not limit the invention.

### Summary of Examples

1. Placebo adhesive for tooth-whitening application
2. Active adhesive for tooth-whitening application (carbamide peroxide)
3. Active adhesive for tooth-whitening application (carbamide peroxide)
4. Active adhesive for tooth-whitening application (carbamide peroxide)
5. Backing Layer Coating Solution (ethyl cellulose:hydroxypropylmethyl cellulose 2:1) plus red dye
6. Backing Layer Coating Solution (ethyl cellulose:hydroxypropylmethyl cellulose 1:1) plus red dye
7. Backing Layer Coating Solution (ethyl cellulose:hydroxypropylmethyl cellulose 2:1)
8. Backing Layer Coating Solution (ethyl cellulose:hydroxypropylmethyl cellulose 1:1)
9. Poly vinyl pyrrolidone laminating solution
10. Coating precast hydroxypropylmethyl cellulose film with backing layer coating solution
11. Placebo composite tooth-whitening device
12. Active composite tooth-whitening device
13. Active composite tooth-whitening device
14. Adhesive only film layer and comparing whitening efficacy against Crest Whitestrips
15. Active composite tooth-whitening device without precast HPMC film
16. Active composite tooth-whitening device
17. Active composite tooth-whitening device without precast HPMC film
18. Placebo composite tooth-whitening device
19. Active composite tooth-whitening device
20. Active composite tooth-whitening device without precast HPMC film
21. Active composite tooth-whitening device
22. Active composite tooth-whitening device without precast HPMC film
23. Active adhesive for tooth-whitening application (sodium percarbonate)
24. Active adhesive for tooth-whitening application (sodium percarbonate)
25. Active adhesive for tooth-whitening application (sodium percarbonate)
26. Active adhesive for tooth-whitening application (sodium percarbonate) plus stabilizers
27. Active adhesive for tooth-whitening application (sodium percarbonate)
28. Active adhesive for tooth-whitening application (sodium percarbonate) plus stabilizers
29. Active adhesive for tooth-whitening application (sodium percarbonate) plus stabilizers and PEG-600
30. Active adhesive for tooth-whitening application (hydrogen peroxide)
31. Active adhesive for tooth-whitening application (hydrogen peroxide) plus stabilizers
32. Active adhesive for tooth-whitening application (PVP/H₂O₂ complex)
33. Active adhesive for tooth-whitening application (PVP/H₂O₂ complex) plus stabilizers
34. Placebo adhesive solution for tooth-bonding layer (hydroxyethyl cellulose)
35. Placebo adhesive solution for tooth-bonding layer (poly acrylic acid)
36. Backing layer coating solution
37. Backing layer film produced on Fortifiber paper
38. Composite tooth-whitening device (adhesive film coated on paper first and backing layer solution coated on top)
39. Composite tooth-whitening device (adhesive film coated on paper first and backing layer solution coated on top)
40. Composite tooth-whitening device (adhesive film coated on paper first and backing layer solution coated on top)
41. Composite tooth-whitening device (backing layer solution coated on paper first and adhesive coated on top)
42. Three layer, composite tooth-whitening device (adhesive tooth bonding layer on paper/active adhesive layer/backing layer film on top)
43. Three layer, composite tooth-whitening device (adhesive tooth bonding layer on paper/active adhesive layer/backing layer film on top)
44. Three layer, composite tooth-whitening device (adhesive tooth bonding layer on paper/active adhesive layer/backing layer film on top)
45. Active mucoadhesive suspension using Amlexanox
46. Active mucoadhesive suspension using Benzocaine
47. Backing layer coating solution (ethyl cellulose: hydroxypropylmethyl cellulose 2:1)
48. Backing layer coating solution (ethyl cellulose: hydroxypropylmethyl cellulose 1:1)
49. Coating precast HPMC film with backing layer coating solution
50. Coating precast HPMC film with backing layer coating solution
51. Preparation of PVP laminating solution
52. Mucoadhesive composite Amlexanox device
53. Mucoadhesive composite Benzocaine device
54. Mucoadhesive composite Benzocaine device
55. Mucoadhesive composite Benzocaine device (reverse laminated, uncoated side of HPMC film is outer layer
56. Active tooth desensitizing adhesive solution
57. Composite tooth desensitizing device
58. Mucoadhesive suspension for an extended breath freshner device
59. Backing layer coating solution (ethyl cellulose:hydroxypropylmethyl cellulose 5:1)
60. Backing layer coating solution (ethyl cellulose:hydroxypropylmethyl cellulose 3:1)
61. Composite breath freshener device

### EXAMPLE 1

A 180.0 gram batch of placebo adhesive solution was prepared using 10.0 grams hydroxyethyl cellulose (Natrosol 250L NF; Hercules), 10.0 grams polyvinyl pyrrolidone (PVP; Povidone P-1416; Spectrum), 0.65 grams sodium benzoate (Spectrum), 0.65 grams propylene glycol (Spectrum), and 158.7 grams deionized and 0.22µ-filtered water. This solution was used in Example 11 below.

### EXAMPLE 2

A 29.87 gram batch of active adhesive solution was prepared using 4.37 grams hydroxyethyl cellulose (Natrosol 250L NF; Hercules), 1.80 grams PVP (Povidone P-1416; Spectrum), 0.09 grams sodium benzoate (Spectrum), 0.09 grams propylene glycol (Spectrum), 2.70 grams carbamide peroxide (Spectrum), and 20.82 grams deionized and 0.22µ-filtered water. This solution was used in Examples 12 and 13 below.

### EXAMPLE 3

A 25.94 gram batch of active adhesive solution was prepared using 3.51 grams hydroxyethyl cellulose (Natrosol 250L NF; Hercules), 1.66 grams PVP (Povidone P-1416; Spectrum), 0.08 grams sodium benzoate (Spectrum), 0.08 grams propylene glycol (Spectrum), 2.49 grams carbamide peroxide (Spectrum), and 18.12 grams deionized and 0.22g-filtered water. This solution was used in Examples 14 and 1.5 below.

### EXAMPLE 4

A 46.84 gram batch of active adhesive solution was prepared using 2.70 grams hydroxyethyl cellulose (Natrosol 250L NF; Hercules), 1.35 grams PVP (Povidone P-1416; Spectrum), 0.15 grams sodium benzoate (Spectrum), 0.15 grams propylene glycol (Spectrum), 2.70 grams carbamide peroxide (Spectrum), 1.95 grams sodium alginate, and 37.84 grams deionized and 0.22µ-filtered water. This solution was used in Examples 16 and 17 below.

### EXAMPLE 5

A 19.0 gm batch of backing solution was prepared using 2.0 grams of ethyl cellulose (Ethocel Premium Std 7; Dow Chemical), 1.0 grams of HPMC (Methocel E5 Prem LV; Dow Chemical), 1.0 gram Adams Extract Red Food Color, and 15.0 grams ethanol (190 proof; USP; Spectrum). This backing solution was used in Examples 11, 15, and 18 below.

### EXAMPLE 6

A 16.5 gram batch of backing solution was prepared using 1.1 grams of ethyl cellulose, 1.1 grams of HPMC, 0.9 grams Adams Extract Red Food Color, and 13.4 grams ethanol (190 proof, USP; Spectrum). This backing solution was used to make Examples 16 and 17 below.

### EXAMPLE 7

A 19.8 gram batch of backing solution was prepared using 2.2 grams of ethyl cellulose, 1.1 grams of HPMC, and 16.5 grams ethanol (190 proof, USP; Spectrum). This backing solution was used to make Examples 12, 13, 19, and 20 below.

### EXAMPLE 8

A 25.2 gram batch of backing solution was prepared using 1.8 grams of ethyl cellulose, 1.8 grams of HPMC, and 21.6 grams ethanol (190 proof, USP; Spectrum). This backing solution was used to make Examples 21 and 22 below.

### EXAMPLE 9

A 52.7 gram batch of laminating solution was prepared using 6.32 grams PVP (P1416; Spectrum), 23.19 grams ethanol (190 proof, USP; Spectrum), and 23.19 grams deionized and 0.22µ filtered water.

### EXAMPLE 10

The precast HPMC film used in several embodiments of this device was typically a 100µ thick sheet called EM1100 from Polymer Films. In some embodiments, it was stretched on a paper and-foil frame of a Werner Mathis AG Lab Coater, type LTF, and a backing solution selected from Examples 5-8 was poured on top and doctor-bladed at a 0.25 mm setting, then dried in the oven section of the Lab Coater.

### EXAMPLE 11

A composite device was made by doctor-blading the adhesive solution of Example 1 into a film using the Lab Coater. The casting was performed on a polymer-coated paper from Fortifiber, which was put on the paper and foil frame of the Lab Coater, with a doctor blade setting of 1.76 mm. The film was automatically dried in the oven portion of the Lab Coater, and a smooth, integral layer of deposited, adhesive polymer resulted. Then, separately, a layer of backing solution from Example 5 was doctor-bladed on top of the precast HPMC film (Example 10) using a 0.25 mm setting. The two films were then laminated together using the laminating solution described in Example 9 and pressure from a roller, followed by drying in the Lab Coater oven. The film was cut either before or after removal from the coated paper and upon application to a moist tooth surface, the film stuck well.

### EXAMPLE 12

A composite device was made as in Example 11, except using the adhesive of Example 2, with doctor-blade setting of 1.00 mm, and the backing solution of Example 7. After cutting and removal from the casting coated paper, the resulting film also stuck well to teeth.

### EXAMPLE 13

Another composite device was made by putting a backing layer of 2:1 ethyl cellulose to HPMC as described in Example 7 on polymer-coated paper (from Fortifiber) and then putting a layer of whitening adhesive (Example 2) on top of it. The backing layer was doctor-bladed at a 0.43 mm setting and the adhesive was doctor-bladed at a setting of 1.30 mm. This film after removal from the paper and cutting, stuck immediately and firmly to teeth, conformed extremely well, and eroded away in about twenty minutes without notice.

### EXAMPLE 14

A composite device composed of only an adhesive layer was made using the process outlined in Example 11, except the adhesive of Example 3 was used and with a doctor blade setting of 1.30 mm. A test was done to determine the whitening efficacy of this adhesive layer alone as compared to the competitive product Crest Whitestrips. Both products were dampened and pressed onto a coffee-stained white cup. They were removed after 16 hours. The amount of whitening was compared and ranked by eight individuals who did not know which device did which whitening. The results shown below indicate that the adhesive layer containing a whitening agent is as effective if not better than one competitive product.

Adhesive only: 5 firsts, 1 tie, and 2 seconds; relative average score = 1.38 Crest White Strip: 2 first, 1 tie, and 5 seconds; relative average score = 1.75

### EXAMPLE 15

A composite device was made by putting a layer of active adhesive from Example 3 on a polymer-coated paper (Fortifiber) and then putting a layer of backing solution from Example 5 on top of it. The adhesive was doctor-bladed at settings of 1.30 mm. The backing layer was spread thinly onto the surface of the dried adhesive film using a spatula and then dried. This resulting film after removal from the coated paper stuck well to the teeth.

### EXAMPLE 16

A composite device was made as in Example 11, except using the adhesive of Example 4 and with a doctor blade setting of 1.30 mm and the backing solution of Example 6 with a doctor blade setting of 0.25 mm. The resulting film after cutting into the desired shape, removal from the coated paper and application to the front teeth, completely eroded in about 1½ hours.

### EXAMPLE 17

A composite device was made as in Example 15, except using the adhesive of Example 4 and the backing solution of Example 6. A strip of this film after removal from the surface of the coated paper and cutting into the desired shape and applied to the front teeth, completely eroded in about 1 hour.

### EXAMPLE 18

A composite device was made as in Example 11, except using the adhesive of Example 3 without the tooth whitening agent, carbamide peroxide, and doctor blade settings of 1.30 mm. After processing, this placebo film visually seemed similar to the active films and upon removal from the coated paper and cutting and application, stuck comparably to the teeth.

### EXAMPLE 19

A composite device was made as in Example 11, except using the adhesive of Example 4, a doctor blade setting of 1.30 mm, and the backing solution of Example 7.

### EXAMPLE 20

A composite device was made as in Example 15, except using the adhesive of Example 4, with a doctor blade setting of 1.30 mm, and the backing solution of Example 7. The resulting film after cutting into the desired shape, removal from the coated paper and application to the front teeth, completely eroded in more than ½ hour.

### EXAMPLE 21

A composite device was made as in Example 11, except using the adhesive of Example 4, with doctor blade setting of 1.30 mm, and the backing solution of Example 8. The resulting film after cutting into the desired shape, removal from the coated paper and application to the front teeth, completely eroded in about 1 ½ hour.

### EXAMPLE 22

A composite device was made as in Example 15, except using the adhesive of Example 4, with doctor blade setting of 1.30 mm, and the backing solution of Example 8. The resulting film after cutting into the desired shape, removal from the coated paper and application to the front teeth, completely eroded in about one hour.

### EXAMPLE 23

A 42.7 gram batch of active adhesive suspension was prepared using 5.00 grams hydroxypropylcellulose (KLUCEL EF PHARM; Hercules), 3.00 grams Povidone K90 USP (P1416, Spectrum), 0.80 grams glycerin (G1016, Spectrum), 0.50 grams Poloxamer 407 (Pluronic F127; P1126, Spectrum), 0.40 grams polyethylene glycol 400 (PEG-8, NF; P0110, Spectrum), 5.00 grams sodium percarbonate (S1601, Spectrum), and 28.0 grams alcohol 190 proof USP (ET108, Spectrum). This suspension was used in Example 37 below.

### EXAMPLE 24

A 46.0 gram batch of active adhesive suspension was prepared using 5.00 grams hydroxypropylcellulose (KLUCEL EF PHARM; Hercules), 3.00 grams Povidone K90 USP (P1416, Spectrum), 0.80 grams glycerin (G1016, Spectrum), 0.50 grams Poloxamer 407 (Pluronic F127; P1126, Spectrum), 0.50 grams polyethylene glycol 400 (PEG-8, NF; P0110, Spectrum), 7.60 grams sodium percarbonate (S1601, Spectrum), and 28.6 grams alcohol 190 proof USP (ET108, Spectrum). This suspension was used in Examples 37, 41 and 43 below.

### EXAMPLE 25

A 196 gram batch of active adhesive suspension was prepared using 22.0 grams hydroxypropylcellulose (KLUCEL EF PHARM; Hercules), 22.0 grams Povidone K90 USP (P1416, Spectrum), 3.20 grams glycerin (G1016, Spectrum), 4.00 grams propylene glycol USP (G1016, Spectrum), 2.00 grams Poloxamer 407 (Pluronic F127; P1126, Spectrum), 1.60 grams polyethylene glycol 400 (PEG-8, NF; P0110, Spectrum), 21.2 grams sodium percarbonate (S1601, Spectrum), and 120 grams alcohol 190 proof USP (ET108, Spectrum). This suspension was used in Examples 38, 40 and 42 below.

### EXAMPLE 26

A 102.2 gram batch of active adhesive suspension was prepared using 11.0 grams hydroxypropylcellulose (KLUCEL EF PHARM; Hercules), 11.0 grams Povidone K90 USP (P-1416, Spectrum), 1.00 grams Povidone K30 USP (P1454, Spectrum), 1.60 grams glycerin (G1016, Spectrum), 2.00 grams propylene glycol USP (G1016, Spectrum), 1.00 grams Poloxamer 407 (Pluronic F127; P1126, Spectrum), 0.80 grams polyethylene glycol 400 (PEG-8, NF; P0110, Spectrum), 10.6 grams sodium percarbonate (S1601, Spectrum), 0.20 grams sodium stannate (S1445, Spectrum), 1.00 gram potassium pyrophosphate (P1462, Spectrum), 4.00 grams deionized and O.22µ-filtered water, and 58.0 grams alcohol 190 proof USP (ET108, Spectrum). This suspension was used in Example 38 below.

### EXAMPLE 27

A 49.1 gram batch of active adhesive suspension was prepared using 5.50 grams hydroxypropylcellulose (KLUCEL EF PHARM; Hercules), 5.50 grams Povidone K90 USP (P-1416, Spectrum), 0.10 grams Carbopol 971 PNF (Novion), 0.80 grams glycerin (G1016, Spectrum), 1.00 grams propylene glycol USP (G1016, Spectrum), 0.50 grams Poloxamer 407 (Pluronic F127; P1126, Spectrum), 0.40 grams polyethylene glycol 400 (PEG-8, NF; P0110, Spectrum), 5.30 grams sodium percarbonate (S1601, Spectrum), and 30.0 grams alcohol 190 proof USP (ET108, Spectrum). This suspension was used in Example 37 below.

### EXAMPLE 28

A 48.2 gram batch of active adhesive suspension was prepared using 5.50 grams hydroxypropylcellulose (KLUCEL EF PHARM; Hercules), 5.50 grams Povidone K90 USP (P-1416, Spectrum), 84 milligrams Carbopol 971 PNF (Novion), 0.80 grams glycerin (G1016, Spectrum), 1.00 grams propylene glycol USP (G1016, Spectrum), 0.50 grams Poloxamer 407 (Pluronic F127; P1126, Spectrum), 0.40 grams polyethylene glycol 400 (PEG-8, NF; P0110, Spectrum), 5.30 grams sodium percarbonate (S1601, Spectrum), 0.10 grams sodium stannate (S1445, Spectrum), 2.50 grams deionized and O.22µ-filtered water, and 26.5 grams alcohol 190 proof USP (ET108, Spectrum). This suspension was used in Example 37 below.

### EXAMPLE 29

A 50.0 gram batch of active adhesive suspension was prepared using 7.00 grams hydroxypropylcellulose (KLUCEL EF PHARM; Hercules), 2.00 grams Povidone K90 USP (P-1416, Spectrum), 5.00 grams Plasdone S-630 (ISP Technologies, Inc), 1.00 grams glycerin (G1016, Spectrum), 1.20 grams propylene glycol USP (G1016, Spectrum), 0.80 grams Poloxamer 407 (Pluronic F127; P1126, Spectrum), 2.00 grams polyethylene glycol 600 (Dow Chemical), 6.00 grams sodium percarbonate (S1601, Spectrum), and 25.0 grams alcohol 190 proof USP (ET108, Spectrum). This suspension was used in Example 39 below.

### EXAMPLE 30

A 35.0 gram batch of active adhesive solution was prepared using 4.00 grams hydroxypropylcellulose (KLUCEL EF PHARM; Hercules), 4.00 grams Povidone K90 USP (P1416, Spectrum), 0.80 grams glycerin (G1016, Spectrum), 0.50 grams Poloxamer 407 (Pluronic F127; P1126, Spectrum), 0.40 grams polyethylene glycol 400 (PEG-8, NF; P0110, Spectrum), 10.0 grams hydrogen peroxide (50%, H1077, Spectrum), and 15.3 grams alcohol 190 proof USP (ET108, Spectrum). This solution was used in Example 38 below.

### EXAMPLE 31

A 180.8 gram batch of active adhesive solution was prepared using 20.0 grams hydroxypropylcellulose (KLUCEL EF PHARM; Hercules), 20.0 grams Povidone K90 USP (P1416, Spectrum), 5.00 grams Poloxamer 407 (Pluronic F127; P1126, Spectrum), 5.00 grams polyethylene glycol 400 (PEG-8, NF; P0110, Spectrum), 42.5 grams hydrogen peroxide (50 CG, Atofina), 0.25 grams edentate disodium USP (ED150, Spectrum), 0.55 grams sodium stennate (S1445, Spectrum), 5.00 grams sodium acid pyrophosphate (S1099, Spectrum), and 82.5 grams alcohol 190 proof USP (ET108, Spectrum). This solution was used in Example 38 below.

### EXAMPLE 32

A 47.5 gram batch of active adhesive solution was prepared using 3.50 grams hydroxypropylcellulose (KLUCEL EF PHARM; Hercules), 6.00 grams Peroxydone K30 (ISP), 7.00 grams Peroxydone K90 (ISP), 1.00 grams Poloxamer 407 (Pluronic F127; P1126, Spectrum), 1.00 grams polyethylene glycol 400 (PEG-8, NF; P0110, Spectrum), and 29.0 grams alcohol 190 proof USP (ET108, Spectrum). This solution was used in Example 38 below.

### EXAMPLE 33

A 208 gram batch of active adhesive solution was prepared using 8.00 grams hydroxypropylcellu lose (KLUCEL EF PHARM; Hercules), 32.0 grams Peroxydone K30 (ISP), 32.0 grams Peroxydone K90 (ISP), 4.00 grams propylene glycol USP (C1016, Spectrum), 4.00 grams Poloxamer 407 (Pluronic F127; P1126, Spectrum), 4.00 grams polyethylene glycol 400 (PEG-8, NF; P0110, Spectrum), 0.20 grams edentate disodium USP (ED150, Spectrum), 0.60 grams sodium stennate (S1445, Spectrum), 4.00 grams sodium acid pyrophosphate (S1099, Spectrum), 20.0 grams deionized and 0.22µ-filtered water, and 99.2 grams alcohol 190 proof USP (ET108, Spectrum). This solution was used in Example 40 below.

### EXAMPLE 34

A 50.0 gram batch of adhesive solution was prepared using 9.0 grams of hydroxyethyl cellulose (Natrosol 250L NF; Hercules), and 41.0 grams deionized and 0.22µ-filtered water. This polymer solution was used in Examples 42 and 43 below.

### EXAMPLE 35

A 50.0 gram batch of adhesive dispersion was prepared using 2.5 grams of Carbopol 980 NF (Noveon), and 47.5 grams alcohol 190 proof USP (ET108, Spectrum). This polymer solution was used in Example 44 below.

### EXAMPLE 36

A 100 gram batch of backing solution was prepared using 10.7 grams of ethyl cellulose (Ethocel Premium Std 7, Dow Chemical), 5.30 grams HPMC (Methocel E5 Prem LV, Dow Chemical), 9 grams peppermint oil NF (PE105, Spectrum), and 75.0 grams alcohol 190 proof USP (ET108, Spectrum). This polymer solution was used in Examples 37, 41, 42, 43, 44 and 57 below.

### EXAMPLE 37

A backing layer film was made by doctor-blading the backing solution of Example 13 using a Werner Mathis AG Lab Coater, type LTF. The casting was performed on a polymer-coated paper from Fortifiber, which was put on the paper and foil frame of the Lab Coater, with a doctor blade setting of 0.92 mm. The film was automatically dried in the oven portion of the Lab Coater, and a thin and homogeneous layer of deposited backing layer film resulted. This backing layer film together with the coated paper was used to make Examples 38, 39 and 40.

### EXAMPLE 38

A composite device was made by doctor-blading the active adhesive suspension or solution selected from Examples 23,24 and 27,31 on the precast backing layer film together with the coated paper obtained from Example 37. The casting was performed using the same Lab Coater with the same manner as in Example 37 with a doctor blade setting of 1.45 mm. The adhesive film was automatically dried in the oven portion of the Lab Coater, and a smooth, integral layer of deposited adhesive polymer film resulted and tightly adhered to the backing layer. This composite film was cut either before or after removal from the coated paper and upon application to a moist tooth surface with the adhesive side, the film stuck well.

### EXAMPLE 39

Similar composite devices were made as in Example 38, except using the active adhesive suspension selected from Example 25, 26, and 29 with doctor blade setting of 1.75 mm. for the first two adhesive suspensions and a setting of 1.70 for the adhesive produced in example 29. After cutting and removal from the casting coated paper, the resulting films stuck well to teeth with better flexibility and mechanical strength. The composite device produced using example 29 adhesive had the most improved characteristics with respect to adhesion, flexibility and mechanical strength.

### EXAMPLE 40

Another similar composite device was made as in Example 39, except using the active adhesive solution from Example 34, with doctor blade setting of 1.60 mm. After cutting and removal from the casting coated paper, the resulting film also stuck well to teeth.

### EXAMPLE 41

A composite device was made by first casting a layer of active adhesive from Example 25 on a polymer-coated paper from Fortifiber using the process outlined in Example 38 with doctor-blade setting of 1.70 mm. Then, a layer of backing solution from Example 36 was coated on top of the dried adhesive film at setting of 1.50 mm. The resulting film also stuck well to teeth.

### EXAMPLE 42

A composite device was made by first casting a layer of polymer adhesive solution from Example 34on a polymer-coated paper from Fortifiber using the process outlined in Example 38 with doctor-blade setting of 1.10 mm. After drying, a layer of active adhesive suspension from Example 2 was coated on top of the resulting HEC film at setting of 1.45 mm. Last, a layer of backing solution from Example 36 was coated on top of the dried active adhesive film at setting of 1.20 mm. The resulting film stuck well to teeth with improved adhesion.

### EXAMPLE 43

A similar composite device was made as in Example 42, except using the active adhesive suspension from Example 25, with doctor blade setting of 1.75 mm, and the backing solution coating at setting of 1.55 mm. The resulting composite film also exhibited good adhesion to teeth.

### EXAMPLE 44

Another similar composite device was made as in Example 42 at the same settings, except using the polymer adhesive dispersion from Example 35. The resulting composite film also stuck well to teeth.

### EXAMPLE 45

A 100 gram batch of active mucoadhesive suspension was prepared using 2.46 grams hydroxyethylcellulose (Natrosol 250LNF, Hercules), 1.25 grams Noveon AA-1 (Noveon), 0.75 grams tragacanth NF (T-300, Importers Service Corporation), 5.10 grams carboxymethylcellulose sodium (7LF PH, Hercules), 0.50 grams propylene glycol USP (PR130, Spectrum), 0.32 grams sodium benzoate NF (SO120, Spectrum), 0.05 grams edetate disodium USP (ED150, Spectrum), 0.20 grams titanium dioxide USP (TI140, Spectrum), 1.30 grams Amlexanox (Takeda), and 88.07 grams deionized and O.22µ-filtered water. This suspension was used in Example 52 below.

### EXAMPLE 46

A 110.0 gram batch of active mucoadhesive suspension was prepared using 4.09 grams hydroxyethylcellulose (Natrosol 250LNF, Hercules), 1.84 grams sodium alginate NF (HF120RBS, FMC BioPolymer), 0.50 grams Noveon AA-1 (Noveon), 1.63 grams tragacanth (T-300, Importers Service Corporation), 1.69 grams povidone K90 USP (P1416, Spectrum), 1.65 grams carboxymethylcellulose sodium (7LF PH, Hercules), 1.02 grams propylene glycol USP (PR130, Spectrum), 0.10 grams methylparaben USP/NF (ME163, Spectrum), 0.05 grams edetate disodium USP (ED150, Spectrum), 0.20 grams titanium dioxide USP (TI140, Spectrum), 11.64 grams benzocaine USP (micronized, Synthesia), and 85.59 grams deionized and O.22µ-filtered water. This suspension was used in Examples 53, 54 and 55 below.

### EXAMPLE 47

A 100 gram batch of backing solution was prepared using 10.73 grams of ethyl cellulose (Ethocel Premium Std 7, Dow Chemical), 5.36 grams HPMC (Methocel E5 Prem LV, Dow Chemical), 0.65 grams propylene glycol USP (G1016, Spectrum), 0.13 grams of FD&C Red #40 powder dye (Sensient Technologies Corporation), 4.5 milligrams of FD&C Blue #1 powder dye (Sensient Technologies Corporation), 2.43 grams deionized and O.22µ-filtered water, and 80.7 grams alcohol 190 proof USP (ET108, Spectrum). This polymer solution was used in Example 49below.

### EXAMPLE 48

A 73.35 gram batch of backing solution was prepared using 5.20 grams of ethyl cellulose (Ethocel Premium Std 7, Dow Chemical), 5.20 grams HPMC (Methocel E5 Prem LV, Dow Chemical), 0.50 grams propylene glycol USP (G1016, Spectrum), 0.05 grams of FD&C Red #40 ALUM Lake (Sensient Technologies Corporation), and 62.4 grams alcohol 190 proof USP (ET108, Spectrum). This polymer solution was used in Example 50 below.

### EXAMPLE 49

The precast HPMC film used in this and in the following examples to produce a variety of mucoadhesive multi-layered devices was typically a 100 µm thick film called EM1100 from Polymer Films. The HPMC film was stretched on a paper-and-foil frame of a Werner Mathis AG Lab Coater, type LTF, and a backing solution of Example 47 was poured on top and doctor-bladed at the setting of 0.25 mm, then dried in the oven section of the Lab Coater. This backing layer film together with the HPMC film was used to make Examples 52 and 53 below.

### EXAMPLE 50

A similar backing layer on the HPMC film was made as in Example 49, except using the backing solution of Example 48. The resulting composite film was used to make Examples 54 and 55 below.

### EXAMPLE 51

A 100 gram batch of laminating solution was prepared dissolving 12 grams povidone K90 USP (P1416, Spectrum) in 44 grams deionized and O.22µ-filtered water and 44 grams alcohol 190 proof USP (ET108, Spectrum). This laminating solution was used to produce multilayered films of different residence times in Examples 52, 53, 54 and 55 below.

### EXAMPLE 52

A composite device was made by doctor-blading the active mucoadhesive suspension of Example 45 into a film using the Lab Coater. The casting was performed on a polymer-coated paper from Fortifiber, which was put on the paper and foil frame of the Lab Coater, with a doctor blade setting of 1.90 mm. The film was automatically dried in the oven portion of the Lab Coater, and a smooth, integral layer of deposited adhesive polymer film resulted. A thin layer of the laminating solution from Example 51 was then applied to the adhesive film; and the adhesive film was laminated to the HPMC/backing layer coating composite film from Example 49 with backing layer coating facing up. Finally, the laminated film was automatically dried in the oven portion of the lab coater. This multilayered film was die cut into ½ inch discs and the discs were stored in heat-sealed aluminum pouches.

### EXAMPLE 53

The experimental process outlined in Example 52 was repeated exactly to make another multilayer film, with the exception that the active adhesive suspension was from Example 46 and the doctor blade setting was at 1.70 mm. This multilayered film was die cut into 9/16 inch discs and the discs were stored in heat-sealed aluminum pouches.

### EXAMPLE 54

A similar multilayer film was made using the same active mucoadhesive suspension and same doctor blade setting as in Example 53, but with the exception that the composite HPMC/backing layer coating film was from Example 49.

### EXAMPLE 55

A similar multilayer film was repeated as Example 54, except that the adhesive film was laminated to the backing coating layer with the HPMC film facing up. The new laminating sequence seemed to improve the adhesive binding efficiency between the layers and the resulting multilayer discs were tested in vivo and yielded a softer mouth feel with comparable residence time.

### EXAMPLE 56

A 88.56 gram batch of tooth-desensitizing adhesive solution was prepared using 11.0 grams hydroxyethyl cellulose (Natrosol 250L NF; Hercules; RM4D08), 11.0 grams PVP (Povidone P-1416; Spectrum; RM3H34), 2.0 grams propylene glycol (Spectrum; RM3B01), 1.6 grams glycerin (USP; Spectrum; RM2L16), 0.8 grams polyethylene glycol (400 Mw; Aldrich, RM4G61), 1.0 gram poloxamer 407 (polyethylene-polypropylene glycol, FCC, P1126; Spectrum; RM0H07), 1.16 grams potassium nitrate (USP, P1843; RM9M30; Spectrum), and 60.0 grams deionized and 0.22g-filtered water. This solution was used in Example 57 below.

### EXAMPLE 57

A composite tooth-desensitizing strip was made by first coating the backing layer solution in Example 35 on polymer-coated paper (from Fortifiber), drying at 50 ° C and then coating a layer of adhesive from Example 56 on top of it and drying at 70 ° C. The backing layer was doctor-bladed at a 1.10 mm setting and the adhesive was doctor-bladed at a setting of 1.90 mm. This film after removal from the paper and cutting, stuck firmly to teeth, conformed extremely well, and eroded away in about one half hour without notice.

### EXAMPLE 58

A 100.2 gram batch of adhesive solution was prepared using 4.67 grams hydroxyethyl cellulose (Natrosol 250L NF; Hercules; RM4D08), 2.61 grams PVP (Povidone P-1416; Spectrum; RM3H34), 3.00 grams propylene glycol (Spectrum), 0.45 grams titanium dioxide (PR130; Spectrum; RM1G09), 1.26 grams sodium alginate (S1118; Spectrum; RM2D44), 1.90 grams tragacanth (TR105; Spectrum; RM2K16), and 89.13 grams deionized and 0.22g filtered water. This solution was used in Example 61 below.

### EXAMPLE 59

A 60.61gram batch of backing solution was prepared using 10.00 grams of ethyl cellulose (Ethocel Std 7 NF; Dow Chemical; RM1M32), 2.00 grams of HPMC (Methocel E5 PREM LV; Dow Chemical; RM1M30), 33.00 grams ethanol (190 proof, USP; Spectrum; RM4D16), 0.60g green food dye (Kroger), and 15.01 grams of peppermint oil (USP, Spectrum; RM4E14). This solution was used in Example 61 below.

### EXAMPLE 60

A 18.07 gram batch of backing solution was prepared using 1.33 grams of HPMC (Methocel E5 PREM LV; Dow Chemical; RM1M30), 4.44 grams of peppermint oil (USP, Spectrum; RM4E14), 0.33g red food dye (Adams Extract Red Food Coloring; RM3A01), and 11.97 grams ethanol (190 proof, USP; Spectrum; RM4D16). This backing solution was also used in the manufacture of Example 61 below.

### EXAMPLE 61

A composite, multilayered breath freshener device was made by doctor-blading the adhesive solution of Example 58 into a film using the Lab Coater. The casting was performed on a polymer-coated paper from Fortifiber, which was put on the paper and foil frame of the Lab Coater, with a doctor blade setting of 2.20 mm. The film was automatically dried in the oven portion of the Lab Coater. A smooth, integral layer of deposited, adhesive polymer resulted. Then, a layer of 5:1 hydrophobic:hydrophilic solution from Example 59 was doctor-bladed on top of the adhesive film using a 1.40 mm setting. This bilayer film was dried in the Lab Coater at 50° C. Lastly, the outer layer solution of Example 60 was spread on top of the 5:1 film. This trilayer film was dried in the Lab Coater at 50° C. The finished film could be cut either before or after removal from the coated paper. A ½" disc was put on the roof of the mouth. The disc stuck well and delivered an initial burst of mint flavor, and a sustained mint flavor for almost 4 hours.

## Claims

1. A water-erodible coating layer for controlling the residence time of a water-soluble thin-film device that is adhered to a moist surface of a body tissue, comprising at least one hydrophobic polymer and at least one water-soluble polymer wherein the ratio of the hydrophobic polymer(s) to the water soluble polymer(s) is from about 1:1 to about 9:1 by weight.

2. The water-erodible coating layer of claim 1, wherein the water-soluble polymer(s) is/are selected from the group consisting of polyvinyl pyrrolidone, polyvinyl alcohol, hydroxyethyl cellulose, hydroxypropyl cellulose and hydroxypropylmethyl cellulose.

3. The water-erodible coating layer of either claim 1 or claim 2, wherein the hydrophobic polymer is selected from the group consisting of methyl cellulose; ethyl cellulose; anionic, cationic and neutral methacrylate polymers and co-polymers (Eudragit^{®} polymers); and a combination thereof.

4. The water-erodible coating layer of claim 1, further comprising a plasticizer.

5. The water-erodible coating layer of claim 4, wherein the plasticizer is selected from the group consisting of propylene glycol, polyethylene glycol and glycerin.

6. The water-erodible coating layer of claim 1, further comprising a humectant.

7. The water-erodible coating layer of claim 6, wherein the humectant is selected from the group consisting of hyaluronic acid and glycolic acid.

8. The water-erodible coating layer of claim 1, further comprising a coloring agent.

9. The water-erodible coating layer of claim 1, further comprising an opacifying agent.

10. The water-erodible coating layer of claim 1, further comprising a flavor or taste-masking agent.

11. A water-erodible thin-film device comprising the water-erodible coating layer of claim 1 and at least one additional layer, wherein the layers are mechanically bonded together and capable of adhering to a moist surface of a body tissue.

12. The device of claim 11, further comprising one or more agents selected from the group consisting of pharmaceutical agents, active ingredients, nutraceuticals, cosmeceuticals, vitamins, botanical extracts, flavors or fragrances are incorporated in one or more layers of the film.

13. The device of claims 11 or 12, wherein the weight of the device is from about 0.01 gram to about 10.0 grams.
